(19) Europäisches Patentamt
European Patent Office
Office européen des brevets

(11) EP 0 752 011 B1

(12) EUROPÄISCHE PATENTSCHRIFT

(45) Veröffentlichungstag und Bekanntmachung des Hinweises auf die Patenterteilung:
**12.05.1999 Patentblatt 1999/19**

(21) Anmeldenummer: **95913108.7**

(22) Anmeldetag: **14.03.1995**

(51) Int Cl.$^6$: **C14C 9/02**

(86) Internationale Anmeldenummer:
**PCT/EP95/00945**

(87) Internationale Veröffentlichungsnummer:
**WO 95/25817 (28.09.1995 Gazette 1995/41)**

# (54) VERWENDUNG VON DIMER- UND/ODER TRIMERAMINPROPIONSÄUREN ZUR FETTENDEN AUSRÜSTUNG VON LEDER

USE OF DIMERIC AND/OR TRIMERIC AMINOPROPIONIC ACIDS FOR OILING OFF OF LEATHERS

UTILISATION D'ACIDES AMINOPROPIONIQUES DIMERES ET TRIMERES POUR METTRE DES CUIRS EN HUILE

(84) Benannte Vertragsstaaten:
**CH DE ES IT LI**

(30) Priorität: **23.03.1994 DE 4409925**

(43) Veröffentlichungstag der Anmeldung:
**08.01.1997 Patentblatt 1997/02**

(73) Patentinhaber: **Henkel Kommanditgesellschaft auf Aktien**
**40191 Düsseldorf (DE)**

(72) Erfinder:
- **ZAUNS-HUBER, Rudolf**
  **D-40589 Düsseldorf (DE)**
- **WOLTER, Fredi**
  **D-41189 Mönchengladbach (DE)**
- **UPHUES, Günter**
  **D-40789 Monheim (DE)**
- **PRINZ, Wolfgang**
  **D-40789 Monheim (DE)**
- **SCHENKER, Gilbert**
  **D-40699 Erkrath (DE)**

(56) Entgegenhaltungen:
**US-A- 3 300 338** **US-A- 3 979 309**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 752 011 B1

## Beschreibung

Die Erfindung betrifft die Verwendung der untenstehend näher bezeichneten Dimer- und/oder Trimeraminpropionsäuren zur fettenden Ausrüstung von Leder. Dabei zeichnen sich diese Verbindungen insbesondere dadurch aus, daß sie den mit ihnen behandelten Ledern bzw. Pelzen gute Wasch- und Reinigungs-Echtheit, Fogging-Echtheit und gute Wasserfestigkeit verleihen.

## Stand der Technik

Die Fettung pflanzlich und/oder mineralisch gegerbter Leder beziehungsweise Pelzfelle ist ein essentieller Verfahrensschritt in der Ausrüstung zum gebrauchsfertigen Wertstoff. Die Form der Fettverteilung in der Hautsubstanz und das Ausmaß der Einbindung der Fettkomponenten in die Hautsubstanz beeinflussen die Eigenschaften und die Gebrauchsfähigkeit der Fertigprodukte entscheidend. Es besteht dabei umfangreiches Fachwissen zu möglichen Interaktionen zwischen den Fettkomponenten einerseits und der gegerbten, sowie Restgerbstoffe enthaltenden Hautsubstanz andererseits. Der spezielle Aufbau der Fettungsmittel - beispielsweise das Ausmaß ihrer lipophilen Gruppen und gegebenenfalls vorliegenden Reaktivgruppen zur Umsetzung mit geeigneten Reaktivbestandteilen im gegerbten Leder - bestimmen unter anderem die Dauerhaftigkeit und Wirkung der fettenden Ausrüstung im praktischen Gebrauch der Leder- und Pelzwaren.

Ein für die Praxis sehr wichtiges Bedürfnis besteht darin, fettende Substanzen bzw. Ausrüstungsmittel zur Verfügung zu stellen, die in der gegerbten Hautsubstanz so zuverlässig gebunden werden können, daß eine für die praktischen Bedürfnisse hinreichende Wasch- und Reinigungsbeständigkeit der Leder- und Pelzwaren sichergestellt ist. Hochwertige Lederwaren, beispielsweise aus der Bekleidungsindustrie, sollen dabei sowohl der wäßrig-tensidischen Wäsche als auch gegebenenfalls einer chemischen Reinigung ohne wesentliche Qualitätseinbuße zugänglich sein.

Für Leder, die im Innenbereich von Autos und Flugzeugen Verwendung finden, besteht darüber hinaus Bedarf, über Substanzen zur fettenden Ausrüstung zu verfügen, die Fogging-echt sind. Unter "Fogging" ist zu verstehen, daß im Laufe der Zeit flüchtige Substanzen aus dem Leder entweichen und sich in unerwünschter Weise niederschlagen, z.B. auf Windschutzscheiben. Unter Fogging-echten Substanzen ist zu verstehen, daß diese Substanzen zum einen selbst so fest im Innern des Leders gebunden sind, daß sie praktisch nicht flüchtig sind, zum anderen, daß diese Substanzen die Fogging-Charakteristik üblicher Fettungsmittel bzw. Fettungsmittelbestandteile verbessern, d.h. deren Fogging-Werte reduzieren.

Schließlich wird für Sonderfälle die weiterführende Bedingung hinreichender Wasserdichtigkeit des fertig ausgerüsteten Leders gewünscht. Zur wasserdichten Ausrüstung von Leder oder Pelzen sind insbesondere drei Verfahrensprinzipien bekannt: (1) Imprägnierung durch Einlagerung wasserunlöslicher Verbindungen, zum Beispiel feste Fette, Wachse oder spezielle Polymere; (2) Imprägnierung durch Einlagerung wasserquellender Verbindungen, die bei Wasseraufnahme hochviskose Emulsionen bilden und die Faserzwischenräume des Leders verstopfen, zum Beispiel spezielle Emulgatoren vom W/O-Typ und (3) Behandlung mit hydrophobierend wirkenden Verbindungen, zum Beispiel Aluminium-, Chrom- und/oder Zirkonkomplexe, Silkone oder organische Fluorverbindungen.

Die DE 1 669 347 beschreibt die Verwendung von wasseremulgierbaren Sulfobernsteinsäure-Halbestern zum Fetten von Leder, wobei jedoch noch keine Wasserdichteffekte erzielt werden.

Gegenstand der EP 193 832 ist ein Verfahren zur Herstellung wasserdichter Leder oder Pelze unter Verwendung von Sulfobernsteinsäure-Monoestern in Kombination mit imprägnierenden und/oder hydrophobierenden Fettungsmitteln.

Die DE 37 17 961 beschreibt ein Verfahren zur Herstellung von N,N-disubstituierten β-Aminopropionsäuren und ihre Verwendung unter anderem zur Hydrophobierung von Leder und Pelzen. Die Herstellung dieser Verbindungen geschieht dabei in zwei Schritten derart, daß zunächst primäre Alkylamine an Acryl- bzw. Methacrylsäure angelagert werden und die dabei entstandenen N-Alkylaminopropionsäuren mit Carbonsäureanhydriden, Carbonsäurechloriden, Sulfonsäurechlorid, Isocyanaten, Halogencarbonsäuren oder Acryl- bzw. Methacylsäuren umgesetzt werden. Die Produkte können gewünschtenfalls anschließend noch - wenigstens teilweise - neutralisiert werden.

In der neueren Patentliteratur werden für die fettende Ausrüstung von insbesondere mineralisch gegerbten Ledern und Pelzen amphiphile Mittel beschrieben, die bestimmt ausgewählte Co-Oligomere von einerseits hydrophoben beziehungsweise oleophilen Monomeren und andererseits hydrophilen Monomerbestandteilen darstellen. Amphiphile Mittel dieser Art können in Form wäßriger Dispersionen, Emulsionen und/oder Lösungen nach Abschluß der Hauptgerbung in die auszurüstenden Leder oder Felle eingearbeitet - beispielsweise eingewalkt - werden. Insbesondere im Fall mineralisch gegerbter Leder oder Pelzfelle können diese amphiphilen Mittel gleichzeitig die Funktion der Nachgerbung übernehmen. Es kann schließlich eine abschließende Fixierung der amphiphilen Mittel mit insbesondere Mineralgerbstoffen vorgesehen sein. Die jüngere Patentliteratur beschreibt Hilfsstoffe der hier betroffenen Art:

So sind zum Beispiel in der EP 372 746 entsprechende Mittel und ihre Anwendung beschrieben, wobei die

amphiphilen Copolymere aus einem überwiegenden Anteil wenigstens eines hydrophoben Monomeren und einem untergeordneten Anteil wenigstens eines copolymerisierbaren hydrophilen Monomeren gebildet sind. Als hydrophobe Monomere sind aufgezählt: langkettige Alkyl(meth)acrylate, langkettige Alkoxy- oder Alkylphenoxy(polyethylen-oxid)-(meth)acrylate, pimäre Alkene, Vinylester von lankettigen Alkylcarbonsäuren und deren Gemische. Die in geringerem Anteil vorliegenden hydrophilen Comonomeren sind ethylenisch ungesättigte wasserlösliche Säuren oder hydrophile basische Comonomere. Das Molekulargewicht (Gewichtsmittel) der Copolymeren liegt im Bereich von 2.000 bis 100.000.

Die EP 412 389 beschreibt als Mittel zum Hydrophobieren von Leder und Pelzfellen den Einsatz von Copolymerisaten, die durch radikalische Copolymerisation von (a) $C_{8-40}$-Monoolefinen mit (b) ethylenisch ungesättigten $C_{4-8}$-Dicarbonsäureanhydriden nach Art einer Substanzpolymerisation bei Temperaturen von 80 bis 300°C zu Copolymerisaten mit Molmassen von 500 bis 20.000 g/Mol, anschließende Solvolyse der Anhydridgruppen der Copolymerisate und zumindest partielle Neutralisation der bei der Solvolyse entstehenden Carboxylgruppen in wäßrigem Medium mit Basen hergestellt worden sind und die in Form von wäßrigen Dispersionen oder Lösungen vorliegen.

In der EP 418 661 wird zum gleichen Zweck die Verwendung von Copolymerisaten beschrieben, die (a) 50 bis 90 Gew.-% $C_{8-40}$-Alkyl(meth)acrylate, Vinylester von $C_{8-40}$-Carbonsäuren oder deren Mischungen und (b) 10 bis 50 Gew.-% monoethylenisch ungesättigte $C_{3-12}$-Carbonsäuren, monoethylenisch ungesättigte Dicarbonsäureanhydride, Halbester oder Halbamide von monoethylenisch ungesättigten $C_{4-12}$-Dicarbonsäuren, Amide von $C_{3-12}$-Monocarbonsäuren oder Mischungen davon einpolymerisiert enthalten und Molmassen von 500 bis 30.000 g/Mol besitzen.

Gegenstand der EP-A-498 632 ist schließlich ein Verfahren zur Herstellung von Leder mit verbesserter Fogging-Charakteristik. Dies wird erreicht durch Behandlung des Leders mit wäßrigen Dispersionen, die frei sind von organischen Lösungsmitteln und die ein amphiphiles Copolymer enthalten, das aus wenigstens einem hydrophilen Monomer und wenigstens einem hydrophoben Monomer besteht.

Schließlich sei darauf hingewiesen, daß es in der Ledertechnik weithin üblich ist, im Anschluß an den Hydrophobierungsschritt eine nachträgliche Fixierung des hydrophobierenden Wirkstoffs durchzuführen. Darunter versteht man einen Verfahrensschritt, durch den sichergestellt werden soll, daß die in das Leder eingetragene hydrophobierende Komponente an die Lederfasern gebunden wird. Üblicherweise wird eine solche Fixierung mit Chrom- und/oder Aluminiumsalzen durchgeführt. Ein Beispiel für diese Technik bietet die DE-A-35 07 241 bzw. die DE-A-36 20 780. In vielen Ländern verschärfen sich jedoch die Auflagen bezüglich des Chromgehalts im Abwasser. Bekanntlich wird aber bei Verwendung konventioneller Gerbstoffe ein nicht unerheblicher Teil von der Lederfaser nicht gebunden und gelangt deshalb über Wasch- und Spülvorgänge ins Abwasser. Beim Einsatz hochauszehrender Chromgerbstoffe ist dieser Anteil zwar geringer, für die Praxis in der Regel aber immer noch auf einem Niveau, das das Bedürfnis nach niedrigeren Werten entstehen läßt.

## Beschreibung der Erfindung

Aufgabe der vorliegenden Erfindung war es, Substanzen für die fettende Ausrüstung von Leder, Pelzen und dergleichen zur Verfügung zu stellen. Dabei sollten diese Substanzen insbesondere in der Lage sein, den mit ihnen behandelten Ledern bzw. Pelzen gute Waschechtheit, Reinigungs-Echtheit und Fogging-Echtheit zu verleihen.

Unter Waschechtheit und Reinigungs-Echtheit ist - wie oben bereits angedeutet und dem Fachmann geläufig - zu verstehen, daß die mit den entsprechenden Substanzen behandelten Leder bzw. Pelze durch die entsprechenden Reinigungsoperationen, z.B. durch das Waschen mit tensidischen Flotten in Waschmaschinen oder die chemische Reinigung in z.B. Perchlorethylen-haltigen Bädern, hinsichtlich ihrer Gebrauchseigenschaften nicht oder nicht wesentlich beeinträchtigt werden, also z.B. nicht schrumpfen.

Eine weitere Aufgabe bestand darin, Substanzen und Verfahrensweisen bereitzustellen, die aus wäßriger Flotte appliziert hydrophobierte Leder ergeben, ohne daß dabei eine nachträgliche Behandlung mit Mineralsalzen durchgeführt werden muß, um die genannte Abwasserkontamination zu vermeiden. Mit anderen Worten bestand Bedarf nach "selbst-fixierenden" Lederhydrophobierungsmitteln.

Die Aufgabe wurde erfindungsgemäß gelöst durch spezielle Dimer- und/oder Trimeraminpropionsäuren (I) der unten näher bezeichneten Struktur.

Gegenstand der vorliegenden Erfindung ist die Verwendung von Dimer- und/oder Trimeraminpropionsäuren (I), die erhältlich sind durch Überführung mindestens einer der in den entsprechenden Dimer- bzw. Trimerfettsäuren enthaltenen Carboxylgruppen ($-CO_2H$) in $-CH_2N[CH_2-CH_2-CO_2H]_2-$ und/oder $-CH_2N[CH_2-CH(CH_3)-CO_2H]_2$-Gruppen, zur fettenden Ausrüstung von Leder. Die Verbindungen (I) können dabei in saurer Form oder in Form ihrer Alkalimetall-, Erdalkalimetall-, Ammonium-, Alkylammonium- oder Alkanolammoniumsalze eingesetzt werden.

Die **Dimer- bzw. Trimeraminpropionsäuren (I)** leiten sich von den literaturbekannten Dimer- bzw. Trimeraminen ab. Ihre Herstellung erfolgt üblicherweise derart, daß man zunächst die in den Dimer- bzw. Trimerfettsäuren enthaltenen Carboxylfunktionen ($-COOH$) in die Aminfunktionen ($-CH_2NH_2$) überführt und die so erhaltenen Amine anschließend durch Umsetzung mit Acryl- und oder Methacyrlsäure funktionalisiert (auf diese Weise werden $-CH_2NH_2$-

Gruppen in Gruppen $-CH_2N[CH_2-CH_2-CO_2H]_2$ bzw. $-CH_2N[CH_2-CH(CH_3)-CO_2H]_2$ überführt).

Das Verhältnis von Dimer- und/oder Trimeramin und Acryl- bzw. Methacrylsäure bei dieser Umsetzung wird dabei so gewählt, daß mindestens eine Aminfunktion ($-CH_2NH_2$) in eine $CH_2N[CH_2-CH_2-CO_2H]_2$- bzw. $-CH_2N[CH_2-CH(CH_3)-CO_2H]_2$-Funktion überführt wird. Dem entspricht in Summe, daß mindestens eine der in den zugrundeliegenden Dimer- bzw. Trimerfettsäuren ursprünglich vorhandenen Carboxylgruppen in eine $CH_2N[CH_2-CH_2-CO_2H]_2$- bzw. $-CH_2N[CH_2-CH(CH_3)-CO_2H]_2$-Gruppe überführt worden ist.

In einer bevorzugten Ausführungsform der vorliegenden Erfindung setzt man solche Verbindungen (I) ein, die erhältlich sind, indem man alle in den entsprechenden Dimer- bzw. Trimerfettsäuren enthaltenen Carboxylgruppen in $CH_2N[CH_2-CH_2-CO_2H]_2$-Gruppen bzw. $-CH_2N[CH_2-CH(CH_3)-CO_2H]_2$-Gruppen überführt.

Unter Dimerfettsäuren sind - wie in der Fachwelt üblich - solche Carbonsäuren zu verstehen, die durch Oligomerisierung ungesättigter Carbonsäuren, in der Regel Fettsäuren wie Ölsäure, Linolsäure, Erucasäure und dergleichen, zugänglich sind. Üblicherweise erfolgt die Oligomerisierung bei erhöhter Temperatur in Gegenwart eines Katalysators aus z.B. Tonerde. Die dabei erhaltenen Produkte stellen Gemische verschiedener Substanzen dar, wobei die Dimersisierungsprodukte überwiegen. Jedoch sind auch geringe Anteile höherer Oligomerer, insbesondere die Trimerfettsäuren, enthalten. Dimerfettsäuren sind handelsübliche Produkte und werden in verschiedenen Zusammensetzungen und Qualitäten angeboten. Im Rahmen der vorliegenden Erfindung sind diejenigen Dimerfettsäuren bevorzugt, die einen Dimergehalt von mindestens 50%, vorzugsweise 75% aufweisen und bei denen die Zahl der C-Atome pro Dimermolekül überwiegend im Bereich von 36 bis 44 liegt.

Analog den Dimerfettsäuren sind auch die Trimerfettsäuren Oligomerisierungsprodukte ungesättigter Fettsäuren, wobei jedoch der Anteil an Trimeren im Produkt überwiegt. Auch Trimerfettsäuren sind handelsübliche Produkte. Bevorzugt sind solche Trimerfettsäuren, bei denen der Trimerengehalt mindestens 50%, vorzugsweise 75% beträgt.

Die Literatur über Dimer- bzw. Trimerfettsäuren ist reichhaltig. Beispielhaft sei in diesem Zusammenhang ein Übersichtsartikel von L.S.Newton zitiert, der eine gut verständliche Einführung in die Chemie der Stammverbindungen und wichtiger Derivate darstellt (vergleiche: Speciality Chemicals 1984 (Mai-Heft), Seiten 17-24). Der Artikel gibt auch ein Beispiel dafür, wie die Transformation der Carboxylfunktionen der Dimer- bzw. Trimerfettsäuren in die entsprechenden Aminfunktionen durchgeführt werden kann, nämlich durch Hydrierung der entsprechenden Nitrile, die ihrerseits durch Dehydrierung der Ammoniumsalze der Säuren zugänglich sind. Die Synthesesequenz läßt sich demnach folgendermaßen veranschaulichen:

a)

$$-CO_2H \text{ (Säure)} \rightarrow -CO_2NH_4 \text{ (Ammoniumsalz)}$$

b)

$$-CO_2NH_4 \text{ (Ammoniumsalz der Säure)} \rightarrow -CN \text{ (Nitril)}$$

c)

$$-CN \text{ (Nitril)} \rightarrow -CH_2NH_2 \text{ (Amin)}$$

In einer bevorzugten Ausführungsform der vorliegenden Erfindung setzt man die Verbindungen (I) in saurer Form oder in Form ihrer Alkalimetall- oder Ammoniumsalze ein.

In einer weiteren Ausführungsform der Erfindung werden die Verbindungen (I) in Kombination mit mindestens einem der nachstehend beschriebenen Aminopropionsäurederivate (II) und/oder Co-Oligomeren (III) eingesetzt.

Die **Aminopropionsäurederivate (II)** sind charakterisiert durch folgende Formel:

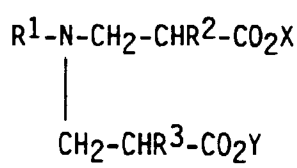

(II)

In der allgemeinen Formel (II) bedeuten

- der Rest $R^1$ eine gesättigte, geradkettige oder verzweigte Alkylgruppe mit 8 bis 22 C-Atomen,
- die Reste $R^2$ und $R^3$ unabhängig voneinander Wasserstoff oder eine Methylgruppe und
- die Reste X und Y unabhängig voneinander Wasserstoff, Alkalimetall, Erdalkalimetall, Ammonium, Alkylammonium oder Alkanolammonium.

Dabei sind diejenigen Verbindungen (II) bevorzugt, in denen der Rest $R^1$ einen geradkettigen Alkylrest mit überwiegend 12 bis 18 C-Atomen, insbesondere mit 12 bis 14 C-Atomen, bedeutet. Die Reste $R^2$ und $R^3$ bedeuten vorzugsweise Wasserstoff. In bezug auf die Reste X und Y gilt, daß diejenigen Verbindungen (I) bevorzugt sind, bei denen einer der Reste X und Y ein Alkalimetall und der andere Wasserstoff bedeutet.

Die **Co-Oligomeren (III)** sind dadurch charakterisiert, daß sie wasserdispergierbares und/oder wasseremulgierbar sind und aufgebaut sind aus

a) Fettcrotonaten und

b) radikalisch copolymerisierbaren hydrophilen ethylenisch ungesättigen Säuren und/oder deren Anhydriden, die auch

c) untergeordnete Mengen weiterer copolymerisierbarer Comonomere enthalten können.

Unter Fettcrotonaten sind dabei Ester der Crotonsäure (trans-2-Butensäure) mit $C_{10-40}$-Fettalkoholen zu verstehen.

Für die Co-Oligomeren (III) gilt, daß diejenigen Fettcrotonat-Bausteine (a) bevorzugt sind, die als Alkohol-Komponente $C_{12-24}$-Fettalkohole enthalten. Geradkettige Fettalkohole bzw. deren Mischungen sind dabei bevorzugt. Es hat sich allerdings gezeigt, daß auch die Mitverwendung von verzweigtkettigen Fettalkoholen im Fettcrotonat-Baustein (a) im Einzelfall zu interessanten Ergebnissen führen kann. So kann beispielsweise durch Mitverwendung von solchen verzweigtkettigen Alkoholen in beschränktem Ausmaß die Penetrationsfähigkeit des Co-Oligomeren (III) in die Faserstruktur des auszurüstenden Hautmaterials gefördert werden. Dabei ist es auch möglich, entsprechende verzweigte Alkohole einer kürzeren C-Zahl einzusetzen, wobei entsprechende Alkohole mit wenigstens 6 C-Atomen, vorzugsweise mit wenigstens 8 C-Atomen, in Betracht kommen. Ein wichtiger verzweigtkettiger Alkohol, der im Zusammenhang mit den Fettcrotonat-Bausteinen (a) zum Einsatz kommen kann, ist das 2-Ethylhexanol. Die Menge der mitzuverwendenden verzweigtkettigen insbesondere kürzeren Alkohole wird allerdings immer vergleichsweise gering sein. So werden in der Regel nicht mehr als 50 Gew.-%, vorzugsweise nicht mehr als 30 oder nicht mehr als 20 Gew.-% der in (a) vorliegenden Alkoholreste durch solche verzweigtkettigen insbesondere niederen Alkohole gebildet. Für das praktische Arbeiten haben sich - bezogen auf das oleophile Fettcrotonat (a) - Mengenverhältnisse von wenigstens etwa 90-95 Gew.-% der ausgeprägt oleophilen langkettigen Fettalkohole, insbesondere des Bereichs $C_{12-18}$, als besonders geeignete Komponenten zur Ausbildung der Crotonatester erwiesen.

Die in den amphiphilen Co-Oligomeren (III) zum Einsatz kommenden hydrophilen Komponenten (b) können den entsprechenden Bausteinen aus den Co-Oligomeren der zitierten EP 372 746, EP 412 389 und EP 418 661 entsprechen. Insbesondere die zuletzt genannte Druckschrift bringt hier ausführliche Angaben zu geeigneten Stoffklassen und zahlreichen speziellen Vertretern.

Besonders wichtige Bausteine (b) für die Co-Oligomeren (III) sind im Sinne der vorliegenden Erfindung ethylenisch ungesättigte Monocarbonsäuren und/oder ethylenisch ungesättigte Dicarbonsäuren und/oder deren Anhydride mit bevorzugt jeweils bis zu 12 C-Atomen. Die Dicarbonsäuren können auch wenigstens anteilsweise in Form ihrer Partialderivate mit einer Carboxylgruppe und einer derivatisierten Carbonsäuregruppe zum Beispiel als Dicarbonsäure-Halbester vorliegen.

Besonders interessante Vertreter aus der Klasse monoethylenisch ungesättigter $C_{3-12}$-Monocarbonsäuren sind beispielsweise Acrylsäure, Methacrylsäure und die Crotonsäure. Besonders geeignete Bausteine (b) können aber auch Dicarbonsäuren beziehungsweise deren Derivate, insbesondere deren Anhydride sein. Typische Vertreter sind die Maleinsäure, Fumarsäure, Itaconsäure, Glutaconsäure und entsprechende Anhydride.

Besondere Bedeutung kommt dabei dem Maleinsäureanhydrid zu. Fettcrotonate (a) und Maleinsäureanhydrid lassen sich in befriedigender Weise mit oder ohne Verwendung von Hilfslösungsmitteln zu Co-Oligomeren (III) eines leicht einstellbar niedrigen Molgewichtes umsetzen. Es ist dabei insbesondere möglich, etwa gleiche Molmengen an Maleinsäureanhydrid und Fettcrotonat miteinander zur Umsetzung zu bringen, so daß nach Solvolyse, insbesondere Hydrolyse der Maleinsäureanhydrid-Bausteine einerseits hinreichend hohe Konzentrationen an Carboxylgruppen für

die Fixierung der Oligomeren in der Haut- beziehungsweise Faserstruktur zur Verfügung stehen, andererseits aber auch zuverlässig hohe Gehalte des oleophilen und damit fettenden und wasserabweisenden Fettcrotonat-Baustein eingebunden sind.

Neben oder anstelle dieser hydrophilen Bausteine (b) auf Basis von Carbonsäuren sind aber auch andere copolymerisierbare hydrophile Verbindungen verwendbar. In Betracht kommen hier insbesondere Sulfosäuregruppen enthaltende ethylenisch ungesättigte Monomere. Bekannte Vertreter dieser Art sind insbesondere entsprechende aliphatische und/oder aromatische Sulfonsäuren, wie Styrolsulfonsäure aber auch Verbindungen wie Vinylsulfonsäure, Allylsulfonsäure, Methallylsulfonsäure und dergleichen. Wie bereits angegeben kann der Baustein (b) des Co-Oligomeren (III) ganz oder teilweise aus solchen der Copolymerisation beziehungsweise Co-Oligomerisation zugänglichen Sulfosäureverbindungen gebildet sein.

Besonders bevorzugt sind solche Co-Oligomere (III) in denen die Bausteine (a) und (b) in Verhältnissen von 30 bis 90 Gew% (a) auf 70 bis 10 Gew.-% (b) vorliegen - Gew.-% hier bezogen auf die Summe von (a) und (b). Bevorzugte Bereiche für die Mischungsverhältnisse sind etwa 35 bis 80 Gew.-% (a) auf 65 bis 20 Gew.-% (b).

Neben den Bausteinen (a) und (b) können die amphiphilen Co-Oligomeren (III) auch untergeordnete Mengen weiterer copolymerisierbarer Comonomere enthalten, die in der obigen Definition als Bausteine (c) bezeichnet sind. Geeignet können hier beliebige der Co-Oligomerisation zugängliche ethylenisch ungesättigte Verbindungen sein, wie sie im einschlägigen Stand der Technik beschrieben sind, vgl. hier beispielsweise EP 418 661 A1, Spalte 3, 49 bis Spalte 4, 27. In der Regel handelt es sich hier um Comonomere, die weder eine ausgeprägte hydrophobierende Wirkung aufweisen noch hydrophilisierende Gruppen im Sinne der Carboxylgruppen oder Sulfonsäuregruppen der erfindungsgemäß mitverwendeten Komponenten (b) besitzen. Werden solche Comonomere (c) in den erfindungsgemäß beschriebenen amphiphilen Co-Oligomeren mitverwendet, so beträgt ihr Anteil vorzugsweise nicht mehr als etwa 30 Gew.-% und insbesondere nicht mehr als etwa 15 Gew.-% - die Angabe Gew.-% - bezieht sich dabei auf die Summe von (a), (b) und (c).

Im Sinne der erfindungsgemäßen Lehre sind diejenigen Co-Oligomere (III) besonders bevorzugt, die frei sind von den gemäß der obigen Definition fakultativen Bausteinen (c), das heißt die ausschließlich aus den Bausteinen (a) und (b) aufgebaut sind. Diese bevorzugten Co-Oligomeren (III) enthalten dabei die Bausteine (a) und (b) in Mischungsverhältnissen von etwa 40 bis 70 Gew.-% (a) auf 60 bis 30 Gew.-% (b); die Gew.-%-Angaben beziehen sich dabei wieder die Summe von (a) und (b).

Die Co-Oligomeren (III) auf Basis der Fettcrotonate weisen bevorzugt mittlere Molgewichte (Molmassen) im Bereich von etwa 500 bis 30.000 g/Mol auf. Geeignet können dabei insbesondere Molmassen im Bereich von etwa 1.000 bis 15.000 g/Mol sein. Es hat sich gezeigt, daß innerhalb dieser vergleichsweise breiten Bereiche den unteren Werten - mittlere Molgewichte beziehungsweise Molmassen im Bereich von etwa 1.000 bis 4.000 oder 5.000 g/Mol und zweckmäßigerweise im Bereich von etwa 1.000 bis 3.000 g/Mol - besondere Bedeutung zukommen kann.

Wie angegeben sind diejenigen Co-Oligomere (III) im Rahmen der erfindungsgemäßen Lehre bevorzugt, die aus der Umsetzung von Fettcrotonaten und Maleinsäureanhydrid resultieren und deren Maleinsäureanhydrid-Bausteine durch Hydrolyse und/oder Solvolyse mit H-aktiven Komponenten in die korrespondierende Form mit freien Carboxylgruppen umgewandelt worden sind. Zur Solvolyse der Maleinsäureanhydrid-Bausteine mit H-aktiven Komponenten eignen sich insbesondere Alkohole aber auch andere Verbindungen wie Carbonsäuren, reaktiven Wasserstoff enthaltende Aminoverbindungen und dergleichen. Durch eine solche gezielte Solvolyse kann gezielt Einfluß genommen werden auf eine Verstärkung der fettenden beziehungsweise wasserabweisenden Eigenschaften der Co-Oligomeren (III). Darüber hinaus kann durch Mitverwendung hinreichend langer Kohlenwasserstoffreste das Verhältnis von oleophilen zu hydrophilen Gruppen in Richtung auf die wasserabweisenden oleophilen Elemente verschoben werden.

Der Zugang zu den Co-Oligomeren (III) ist aber auch beispielsweise auf folgende Art möglich: In einer ersten Verfahrensstufe werden die freie Crotonsäure und Maleinsäureanhydrid miteinander zur entsprechenden Oligomerverbindung umgesetzt. Anschließend wird unter Ausschluß von Wasser und raschem Austrag des Kondensationswassers eine Veresterung der Crotonsäurebestandteile mit den gewünschten Fettalkoholen beziehungsweise Fettalkoholgemischen vorgenommen. Nachfolgend wird dann die Hydrolyse beziehungsweise Solvolyse der Anhydridringe durchgeführt.

Die **Verbindungen (I)** sind zur raschen und durchdringenden imprägnierenden Ausrüstung von Leder und/oder Pelzen besonders geeignet, so daß Leder zugänglich werden, die sich durch ihre Waschechtheit und auch Reinigungsechtheit (z.B. in bezug auf eine chemische Reinigung) auszeichnen. Sie sind darin ähnlichen Verbindungen, die aus dem Stand der Technik bekannt sind, überlegen. Die erfindungsgemäßen Verbindungen (I) zeichnen sich darüber hinaus durch gute Fogging-Echtheit sowie durchgute hydrophobierende Eigenschaften (Erhöhung der Wasserdichtigkeit) aus. Ein weiterer Vorteil der Verbindungen (I) besteht darin, daß sie selbst-fixierend sind, d.h. daß im Anschluß an den Eintrag des fettenden bzw. hydrophobierenden Wirkstoffs (I) in das Leder ein anschließender zusätzlicher Fixierungsschritt nicht zwingend erforderlich ist.

Die Verbindungen (I) werden üblicherweise in wäßrigem Milieu zur fettenden Ausrüstung von Leder, insbesondere chromgegerbtem Leder, eingesetzt. Dabei kann es sich - je nach der Natur der Reste $M^1$ bis $M^4$ beziehungs-

weise der - gegebenenfalls substituierten Alkylengruppe [-$(CH_2)_x$-] um wäßrige Lösungen oder um wäßrige Dispersionen der Verbindungen (I) handeln.

Ein weiterer Gegenstand der vorliegenden Erfindung sind daher Mittel zur fettenden Ausrüstung von Ledern, Pelzen und dergleichen in Form einer wäßrigen Lösung bzw. Dispersion, die sich durch einen Gehalt an Dimer- und/ oder Trimeraminpropionsäuren (I) auszeichnen.

Diese **wäßrigen Angebotsformen** werden bevorzugt auf einen schwach sauren bis schwach alkalischen Bereich eingestellt. Zur Sicherstellung der Lagerstabilität kann es dabei zweckmäßig sein, Angebotsformen vorzusehen, deren wäßrige Phase durch Zugabe von anorganischen und/oder organischen Basen auf neutrale bis schwach alkalische pH-Werte eingestellt ist. Zur pH-Regulierung eignen sich an sich alle im einschlägigen Stand der Technik beschriebenen Basen. Besonders bevorzugt sind die Alkalisalze, insbesondere des Natriums und/oder Kaliums. Aber auch Ammoniumsalze oder Salze von Alkanolaminen wie Diethanolamin sind geeignete Vertreter. Bevorzugt werden die pH-Werte der wäßrigen Angebotsformen der Verbindungen (I) - oder deren Kombinationen mit den Verbindungen (II) und/oder (III) - im Bereich um pH 7 bis 8 eingestellt.

Der Wertstoffgehalt der wäßrigen Angebotsformen an den Verbindungen (I) - oder deren Kombinationen mit den Verbindungen (II) und/oder (III) - wird typischerweise im Bereich von etwa 20 bis 75 Gew.-% eingestellt. Sie lassen sich mit Wasser und/oder wäßrigen Wirkstoffabmischungen der nachfolgend geschilderten Art jederzeit aufmischen und zum praktischen Einsatz bringen.

Die Verbindungen (I) - sowie auch deren Kombinationen mit den Verbindungen (II) und/oder (III) - zeichnen sich durch selbstemulgierende Eigenschaften aus, so daß für den praktischen Einsatz der wäßrigen Angebotsformen die Anwesenheit weiterer Emulgatoren an sich entbehrlich ist.

Es kann aber gewünscht sein, daß die wäßrigen Angebotsformen der Verbindungen (I) - beziehungsweise deren Kombinationen mit den Verbindungen (II) und/oder (III) - weitere ausgewählte Emulgatoren enthalten, die beim Eintrag in insbesondere mineralgegerbte Leder und/oder Felle eine zusätzliche Fettung beziehungsweise Hydrophobierung bewirken und bevorzugt gleichzeitig über saure Gruppen im gegerbten Leder beziehungsweise Fell fixiert werden können. Ein wichtiges Beispiel für Verbindungen dieser Art sind die eingangs genannten in Wasser emulgierbaren Sulfobernsteinsäure-Halbester, die von langkettigen Fettalkoholen und/oder ihren Alkylenoxidaddukten abstammen. Ein wichtiges Beispiel für die Emulgatorenklasse der hier betroffenen Art sind $C_{18}$-Sulfobernsteinsäure-Halbester. Es hat sich gezeigt, daß durch die Mitverwendung solcher emulgatorartiger Hilfskomponenten - die an sich als Ausrüstungsmittel für die Lederfettung vorbekannt sind - im Sinne der erfindungsgemäßen Zielsetzung vorteilhafte Wirkungen erreicht werden können. Als Beispiele für Verbindungstypen dieser Art seien im einzelnen hier benannt:

Sulfobernsteinsäure-Halbester langkettiger Fettalkohole mit insbesondere 12 bis 24 C-Atomen und/oder deren Alkylenoxidaddukten mit bevorzugt bis zu 6 Alkylenoxidresten, entsprechende Sulfobernsteinsäure-Halbester von Fettsäuremono- und/oder -diglyceriden beziehungsweise deren Alkylenoxidaddukten mit bevorzugt bis zu 6 Alkylenoxidresten bei weiterhin bevorzugter Kettenlänge der Fettsäure(n) im Bereich $C_{12-24}$, langkettige Sulfofettsäuren, insbesondere entsprechende alpha-Sulfofettsäuren mit vorzugsweise 12 bis 24, insbesondere 16 bis 18 C-Atomen, wobei im Falle dieser alpha-substituierten Sulfofettsäuren die Kohlenwasserstoffreste üblicherweise gesättigt sind, sowie innenständige Sulfofettsäuren von olefinisch 1- und/oder mehrfach ungesättigten Carbonsäuren wie Ölsäure, Linolsäure, Linolensäure und dergleichen.

Die wäßrigen Angebotsformen der Verbindungen (I) - sowie auch deren Kombinationen mit den Verbindungen (II) und/oder (III) - können auch weitere Fettungs- beziehungsweise Hydrophobierungsmittel im Sinne der Wertstoffgemische enthalten, wie sie in der eingangs zitierten EP 193 832 beschrieben sind. In dieser Ausführungsform der Erfindung werden die Verbindungen (I) kombiniert mit imprägnierenden und/oder hydrophobierenden Fettungsmitteln, wie Sulfobernsteinsäuremonoester-Salze mit $C_{12-24}$-Fettresten, in Kombination mit weiteren imprägnierenden Fettungsmitteln, ausgewählt insbesondere aus der Gruppe der oxidierten oder oxidierten und teilsulfierten $C_{18-26}$-Kohlenwasserstoffe oder $C_{32-40}$-Wachse eingesetzt. Andere Beispiele für diese zusätzlichen imprägnierenden Fettungsmittel sind Phosphorsäuremono-$C_{12-24}$-Alkylester, Partialester von Polycarbonsäuren wie Citronensäure-Mono-$C_{16-24}$-Alkylester, Partialester von Polyalkoholen wie Sorbitan-, Glycerin- oder Pentaerythrit-$C_{16-24}$-fettsäureester.

Eine besonders geeignete Emulgatorklasse, die im Rahmen der erfindungsgemäßen Lehre mitverwendet werden kann, sind die aus der Ausrüstung von Ledern und Pelzen mit Fettstoffen bekannten N-Acylaminosäuren, insbesondere Fettsäuresarkoside, z.B. N-Oleoyl-sarkosin, wie sie beispielsweise als Emulgatoren zum Eintrag von Silikonölen in Leder und Pelze in der EP-B-213 480 im einzelnen beschrieben sind. Geeignete Emulgatoren sind demgemäß insbesondere Salze von N-($C_{9-20}$-Acyl)-aminosäuren, wobei entsprechenden Salzen einer Aminosäure mit 2 bis 6 C-Atomen, die mit dem Acylrest einer gesättigten oder ungesättigten Fettsäure mit 9 bis 20 C-Atomen am Aminstickstoff, der gegebenenfalls zusätzlich durch Methyl substituiert ist, besondere Bedeutung zukommt. Geeignete Salze dieser Emulgatoren sind wiederum insbesondere Alkali-, Ammonium- oder Alkanolaminsalze.

Von den N-($C_{9-20}$-Acyl)-aminosäuren sind solche mit 2 bis 4 C-Atomen und mit der Aminogruppe in alpha-Stellung zur Carboxylgruppe besonders bevorzugt, die weiterhin am Aminstickstoffatom zusätzlich durch eine Methylgruppe substituiert sind. Davon weisen eine besonders überlegene Wirkung die Fettsäuresarkoside von gesättigten

oder ungesättigten Fettsäuren mit 9 bis 20, bevorzugt 16 bis 18 C-Atomen auf. Das bevorzugte Sarkosid ist das Ölsäuresarkosid. Weiterhin sind insbesondere das N-Stearoyl-sarkosin, N-Lauroyl-sarkosin und N-Isononanoyl-sarkosin besonders geeignet und zwar jeweils in Form ihrer Alkalisalze, Ammoniumsalze oder der Salze von Mono-, Di- oder Trialkanolaminen mit insbesondere 2 bis 4 C-Atomen im Alkanolrest.

Beim Einsatz von solchen Wertstoffgemischen beträgt die Menge der erfindungsgemäß einzusetzenden Verbindungen (I) - beziehungsweise die Gesamtmenge der Verbindungen (I) bis (III) - bevorzugt wenigstens etwa 35 Gew.-% des Wertstoffgemisches und insbesondere wenigstens etwa 50 Gew.-%. Es kann jedoch zweckmäßig sein, wenigstens etwa 70 bis 80 Gew.-% des insgesamt in die auszurüstenden Leder beziehungsweise Pelzfelle einzubringenden Wertstoffgemisches auf Basis der Verbindungen (I) vorzusehen.

Der Eintrag der erfindungsgemäßen Verbindungen (I) - beziehungsweise deren Kombinationen mit den Verbindungen (II) und/oder (III) - über ihre wäßrige Angebotsform beziehungsweise ihrer Abmischungen mit den genannten weiteren Komponenten erfolgt in an sich bekannter Weise - siehe hierzu insbesondere auch die Angaben der eingangs genannten Druckschriften. Nur kurz sei daher zusammenfassend dargestellt:

Die erfindungsgemäßen Verbindungen (I) - beziehungsweise deren Kombinationen mit den Verbindungen (II) und/oder (III) - eigenen sich zur Behandlung von allen üblichen gegerbten Häuten, insbesondere entsprechendem Material, das mit Mineralgerbstoffen gegerbt worden ist. Die gegerbten Häute werden üblicherweise vor der Behandlung entsäuert. Sie können bereits vor der Behandlung gefärbt worden sein. Eine Färbung kann aber auch erst nach der erfindungsgemäß durchgeführten Behandlung vorgenommen werden.

Das zur imprägnierende Leder wird üblicherweise mit den Verbindungen (I) - beziehungsweise deren Kombinationen mit den Verbindungen (II) und/oder (III) - in wäßriger Flotte zweckmäßig bei pH-Werten von etwa 4 bis 10 und vorzugsweise bei pH 5 bis 8 und bei Temperaturen von etwa 20 bis 60°C, vorzugsweise 30 bis 50°C, während eines Zeitraumes bis zu einigen Stunden gegebenenfalls mehrstufig behandelt. Die Behandlung erfolgt beispielsweise durch Walken in einem Faß. Die Menge an der erfindungsgemäßen Verbindung (I) - beziehungsweise die Gesamtmenge der Verbindungen (I) bis (III) - in Form ihrer wäßrigen Angebotsform beträgt üblicherweise 0,1 bis 30 Gew.-%, insbesondere 1 bis 20 Gew.-% bezogen auf das Falzgewicht des Leders oder das Naßgewicht der Pelzfelle. Die Flottenlänge beträgt üblicherweise 10 bis 1.000 %, vorzugsweise 30 bis 150 %, bei Pelzfellen 50 bis 500 %.

Nach Abschluß der Behandlung mit der wäßrigen Flotte wird der pH-Wert der Behandlungsflotte durch Zusatz von Säuren in den leicht sauren Bereich verschoben. Geeignet ist insbesondere der Zusatz organischer Säuren, bevorzugt Ameisensäure. Bevorzugte pH-Werte liegen im Bereich von 3 bis 5, vorzugsweise im Bereich von etwa 3,5 bis 4.

Gewünschtenfalls kann eine Fixierung mit insbesondere Mineralgerbstoffen nachgeschaltet werden, wobei hier der Einsatz von Aluminiumsalzen, aber auch von anderen mehrwertigen Mineralsalzen, z.B. Chrom- oder Zirkonsalzen besonders bevorzugt sein kann. Wie bereits ausgeführt ist eine derartige Fixierung aber nicht zwingend erforderlich.

Ein weiterer Gegenstand der vorliegenden Erfindung ist daher ein Verfahren zur Hydrophobierung von Leder, Pelzen und dergleichen unter Verwendung von Dimer- und/oder Trimeraminpropionsäuren (I), wobei man diese Verbindungen in saurer Form oder in Form ihrer Alkalimetall-, Erdalkalimetall-, Ammonium-, Alkylammonium- oder Alkanolammoniumsalze einsetzt, wobei man Leder bzw. Pelze in wäßriger Flotte mit Hydrophobierungsmitteln behandelt, die mindestens eine der Verbindungen (I) enthält, wobei man auf eine spätere Mineralsalzfixierung verzichtet.

Beispiele

**1. Herstellung der Verbindungen (I)**

Die Verbindungen (I) können - wie oben angegeben - nach literaturbekannten Methoden hergestellt werden. Der Schlüsselschritt besteht dabei in der Umsetzung der Aminogruppen-funktionalisierten Vorstufe mit Acryl- und/oder Methacrylsäure. Die Durchführung dieses letztgenannten Reaktionstyps sei beispielhaft anhand der Umsetzung von 1,6-Diaminohexan mit Acrylsäure beschrieben:

In einen Dreihalskolben mit regelbarem Innenthermometer, Tropftrichter und einem Stickstoff-Überleitungsrohr wurden 622 g Wasser sowie 194,6 g (2,70 Mol) Acrylsäure vorgelegt. Anschließend wurden langsam 108,0 g (1,35 Mol) einer 50 gew.-%igen wäßrigen Natronlauge innerhalb von 10 Minuten zugetropft. Die Temperatur der Reaktionsmischung stieg dabei auf 50 °C. Man erhitzte auf 60 °C und tropfte 75,7 g (0,67 Mol) 1,6-Diaminohexan innerhalb von 10 Minuten zu. Die dabei stattfindende Reaktion war begleitet von einer starken nebelartigen Rauchentwicklung und einem Temperaturanstieg auf 80 °C. Man erhitzte anschließend auf eine Temperatur von 90 °C und rührte die Reaktionsmischung 5 Stunden bei dieser Temperatur. Isoliert wurden 992 g einer leicht gelblich gefärbten Flüssigkeit mit folgenden Kennzahlen: Aminzahl = 70,0; Säurezahl = 74,5; Wasserwert (nach Fischer) = 68 %.

### 2. Ausführungsbeispiele

Waschechtheit, Reinigungsechtheit, Fogging-Echtheit und Wasserdichtigkeit von Ledern, die mit den Verbindungen (I) behandelt worden waren, wurden generell als gut eingestuft.

Die Wasserdichtigkeit von Ledern, die mit den Verbindungen (I) behandelt worden waren, wurde dabei - als Maß für die hydrophobierenden Eigenschaften der Verbindungen (I) - mit einem Balley-Penetrometer gemäß Meßmethode IUP der Internationalen Union der Leder-Chemiker-Verbände (Kommission für physikalische Lederprüfung) untersucht. Die Methode ist publiziert in: Das Leder, 1961, 12. Jahrgang, Seiten 36-40. Als Kenngröße für die Güte der Hydrophobierung diente dabei die Wasserdurchtrittszeit. Die Stauchung der Leder betrug bei diesen Untersuchungen 15%.

Es stellte sich heraus, daß die erfindungsgemäß einzusetzenden Verbindungen (I) gute bis sehr gute Werte in Bezug auf die Wasserdurchtrittszeiten aufwiesen. Dies ist umso bemerkenswerter, als die Stauchung der Leder bei den Penetrometertests auf 15% eingestellt wurde, was bedeutet, daß die Lederfasern unter diesen Prüfbedingungen sehr hohen Stauchungskräften ausgesetzt waren.

### Patentansprüche

**1.** Verwendung von Dimer- und/oder Trimeraminpropionsäuren (I), erhältlich durch Überführung mindestens einer der in den entsprechenden Dimer- bzw. Trimerfettsäuren enthaltenen Carboxylgruppen in $-CH_2N[CH_2-CH_2-CO_2H]_2-$ und/oder $-CH_2N[CH_2-CH(CH_3)-CO_2H]_2$ Gruppen, wobei man die Verbindungen (I) in saurer Form oder in Form ihrer Alkalimetall-, Erdalkalimetall-, Ammonium-, Alkylammonium- oder Alkanolammoniumsalze einsetzt, zur fettenden Ausrüstung von Leder.

**2.** Verwendung nach Anspruch 1, wobei man solche Verbindungen (I) einsetzt, die durch Überführung aller in den entsprechenden Dimer- bzw. Trimerfettsäuren enthaltenen Carboxylgruppen ($-CO_2H$) in $-CH_2N[CH_2-CH_2-CO_2H]_2-$ und/oder-$CH_2N[CH_2-CH(CH_3)-CO_2H]_2$-Gruppen erhalten wurden.

**3.** Verwendung nach Anspruch 1 oder 2, wobei man Verbindungen (I) in Kombination mit

i) mindestens einem Aminopropionsäurederivat der allgemeinen Formel (II)

$$\begin{array}{l} R^1-N-CH_2-CHR^2-CO_2X \\ \quad\;\; | \\ \quad\;\; CH_2-CHR^3-CO_2Y \end{array} \qquad (II)$$

worin

- der Rest $R^1$ eine gesättigte, geradkettige oder verzweigte Alkylgruppe mit 8 bis 22 C-Atomen,
- die Reste $R^2$ und $R^3$ unabhängig voneinander Wasserstoff oder eine Methylgruppe und
- die Reste X und Y unabhängig voneinander Wasserstoff, Alkalimetall, Erdalkalimetall, Ammonium, Alkylammonium oder Alkanolammonium bedeuten

und /oder

ii) mindestens einem wasserdispergierbaren und/oder wasseremulgierbaren Co-Oligomeren (III) aus

a) Fettcrotonaten und

b) radikalisch copolymerisierbaren hydrophilen ethylenisch ungesättigten Säuren und/oder deren Anhydriden, die auch

c) untergeordnete Mengen weiterer copolymerisierbarer Comonomere enthalten können,

einsetzt.

**4.** Verwendung nach einem der Ansprüche 1 bis 3, wobei man die Verbindungen (I) - beziehungsweise deren Kombination mit mindestens einer der Verbindungen (II) und/oder (III) - in wäßriger Angebotsform, d.h. in Form einer wäßrigen Lösung bzw. Dispersion, einsetzt.

**5.** Verwendung nach Anspruch 4, wobei man den pH-Wert der wäßrigen Angebotsform im Bereich von schwach sauer bis schwach alkalisch einstellt.

**6.** Verfahren zur Hydrophobierung von Leder, Pelzen und dergleichen unter Verwendung von Dimer- und/oder Trimeraminpropionsäuren (I), wobei man diese Verbindungen in saurer Form oder in Form ihrer Alkalimetall-, Erdalkalimetall-, Ammonium-, Alkylammonium- oder Alkanolammoniumsalze einsetzt, wobei man Leder bzw. Pelze in wäßriger Flotte mit Hydrophobierungsmitteln behandelt, die mindestens eine der Verbindungen (I) enthält, die erhältlich sind durch Überführung mindestens einer der in den entsprechenden Dimer- bzw. Trimerfettsäuren enthaltenen Carboxylgruppen in $-CH_2N[CH_2-CH_2-CO_2H]_2$- und/oder $-CH_2N[CH_2-CH(CH_3)-CO_2H]_2$Gruppen, wobei man die Verbindungen (I) in saurer Form oder in Form ihrer Alkalimetall-, Erdalkalimetall-, Ammonium-, Alkylammonium- oder Alkanolammoniumsalze einsetzt, dadurch gekennzeichnet, daß man auf eine spätere Mineralsalzfixierung verzichtet.

**7.** Mittel zur fettenden Ausrüstung von Ledern, Pelzen und dergleichen in Form einer wäßrigen Lösung oder Dispersion, dadurch gekennzeichnet, daß sie Dimer- und/oder Trimeraminpropionsäuren (I) enthalten, die erhältlich sind durch Überführung mindestens einer der in den entsprechenden Dimer- bzw. Trimerfettsäuren enthaltenen Carboxylgruppen ($-CO_2H$) in $-CH_2N[CH_2-CH_2-CO_2H]_2$- und/oder $-CH_2N[CH_2-CH(CH_3)-CO_2H]_2$-Gruppen, wobei man die Verbindungen (I) in saurer Form oder in Form ihrer Alkalimetall-, Erdalkalimetall-, Ammonium-, Alkylammonium- oder Alkanolammoniumsalze einsetzt.

## Claims

**1.** The use of dimer and/or trimer aminopropionic acids (I) obtainable by converting at least one of the carboxyl groups present in the corresponding dimer or trimer fatty acids into $-CH_2N[CH_2-CH_2-CO_2H]_2$ and/or $-CH_2N[CH_2-CH(CH_3)-CO_2H]_2$ groups, the compounds (I) being used in acidic form or in the form of their alkali metal, alkaline earth metal, ammonium, alkylammonium or alkanolammonium salts, for the oiling of leather.

**2.** The use claimed in claim 1, characterized in that compounds (I) obtained by converting all the carboxyl groups ($-CO_2H$) present in the corresponding dimer or trimer fatty acids into $-CH_2N[CH_2-CH_2-CO_2H]_2$ and/or $-CH_2N[CH_2-CH(CH_3)-CO_2H]_2$ groups are used.

**3.** The use claimed in claim 1 or 2, characterized in that compounds corresponding to formula (I) are used in combination with

i) at least one aminopropionic acid derivative corresponding to general formula (II):

$$\begin{array}{l} R^1\text{-}N\text{-}CH_2\text{-}CHR^2\text{-}CO_2X \\ \quad\;\; | \\ \quad\;\; CH_2\text{-}CHR^3\text{-}CO_2Y \end{array} \qquad (II)$$

in which

- $R^1$ is a saturated, linear or branched alkyl group containing 8 to 22 carbon atoms,
- $R^2$ and $R^3$ independently of one another represent hydrogen or a methyl group and
- X and Y independently of one another represent hydrogen, alkali metal, alkaline earth metal, ammonium, alkylammonium or alkanolammonium

and/or

ii) at least one water-dispersible and/or water-emulsifiable co-oligomer (III) of

a) fatty crotonates and
b) radical-copolymerizable hydrophilic, ethylenically unsaturated acids and/or anhydrides thereof which may also contain
c) small quantities of other copolymerizable comonomers.

**4.** The use claimed in any of claims 1 to 3, characterized in that the compounds (I) - or combinations thereof with at least one of the compounds (II) and/or (III) - are used in the form of an aqueous formulation, i.e. in the form of an aqueous solution or dispersion.

**5.** The use claimed in claim 4, characterized in that the aqueous formulation is adjusted to a mildly acidic to mildly alkaline pH value.

**6.** A process for hydrophobicizing leather, skins and the like using dimer and/or trimer aminopropionic acids (I) in acidic form or in the form of their alkali metal, alkaline earth metal, ammonium, alkylammonium or alkanolammonium salts, leather or skins being treated in aqueous medium with hydrophobicizing agents containing at least one of the compounds (I) obtainable by converting at least one of the carboxyl groups present in the corresponding dimer or trimer fatty acids into $-CH_2N[CH_2-CH_2-CO_2H]_2$ or $-CH_2N[CH_2-CH(CH_3)-CO_2H]_2$ groups, the compounds (I) being used in acidic form or in the form of their alkali metal, alkaline earth metal, ammonium, alkylammonium or alkanolammonium salts, characterized in that the hydrophobicizing agents are not subsequently fixed with mineral salts.

**7.** Formulations for oiling leathers, skins and the like in the form of an aqueous solution or dispersion, characterized in that they contain dimer and/or trimer aminopropionic acids (I) obtainable by converting at least one of the carboxyl groups ($-CO_2H$) present in the corresponding dimer or trimer fatty acids into $-CH_2N[CH_2-CH_2-CO_2H]_2$ and/or $-CH_2N[CH_2-CH(CH_3)-CO_2H]_2$ groups, the compounds (I) being used in acidic form or in the form of their alkali metal, alkaline earth metal, ammonium, alkylammonium or alkanolammonium salts.

## Revendications

**1.** Utilisation d'acides aminopropioniques diméres et/ou trimères (I), obtenables par transformation d'au moins un des groupes carboxyle contenus dans les acides gras dimères ou triméres correspondants, en groupes $-CH_2N[CH_2-CH_2-CO_2H]_2$ et/ou $-CH_2N[CH_2-CH(CH_3)-CO_2H]_2$, dans laquelle on met en oeuvre les composés (I) sous forme d'acide ou de leurs sels de métaux alcalins, de métaux alcalino-terreux, d'ammonium, d'alkylammonium ou d'alcanolammonium, pour mettre les cuirs en huile.

**2.** Utilisation selon la revendication 1, dans laquelle on met en oeuvre les composés (I), qui ont été obtenus par transformation de tous les groupes carboxyle ($-CO_2H$) contenus dans les acides gras dimères ou trimères correspondants, en groupes $-CH_2N[CH_2-CH_2-CO_2H]_2$ et/ou $-CH_2N[CH_2-CH(CH_3)-CO_2H]_2$.

**3.** Utilisation selon la revendication 1 ou 2, dans laquelle on met en oeuvre les composés (I) en association avec

i) au moins un dérivé d'acide aminopropionique de la formule générale (II)

$$\begin{array}{l} R^1-N-CH_2-CHR^2-CO_2X \\ \quad\;\; | \\ \quad\;\; CH_2-CHR^3-CO_2Y \end{array} \qquad (II)$$

dans laquelle

- le radical $R^1$ représente un groupe alkyle saturé, à chaîne droite ou ramifiée, comportant 8 à 22 atomes de C,

- les radicaux $R^2$ et $R^3$ correspondent indépendamment l'un de l'autre à l'hydrogène ou à un groupe méthyle et
- les radicaux X et Y sont indépendamment l'un de l'autre l'hydrogène, un métal alcalin, un métal alcalino-terreux, un ammonium, un alkylammonium ou un alcanolammonium

et/ou

ii) au moins un co-oligomère dispersible et/ou émulsionnable dans l'eau (III) à base

a) de crotonates gras et

b) d'acides à insaturation éthylénique, hydrophiles, copolymérisables par voie radicalaire et/ou de leurs anhydrides, qui peuvent également renfermer

c) des quantités secondaires d'autres comonoméres copolymérisables.

**4.** Utilisation selon les revendications 1 à 3, dans laquelle on met en oeuvre les composés (I) - ou leur association avec au moins un des composés (II) et/ou (III) - en présentation aqueuse, c'est-à-dire sous la forme d'une solution ou d'une dispersion aqueuse.

**5.** Utilisation selon la revendication 4, dans laquelle on ajuste le pH de la présentation aqueuse dans l'intervalle de légèrement acide à légèrement alcalin.

**6.** Procédé d'imperméabilisation du cuir, des peaux et des matières similaires, en utilisant des acides aminopropioniques diméres et/ou trimères (I), dans lequel on met en oeuvre ces composés sous forme d'acide ou de leurs sels de métaux alcalins, de métaux alcalino-terreux, d'ammonium, d'alkylammonium ou d'alcanolammonium, en traitant les cuirs ou les peaux dans un bain aqueux par un agent imperméabilisant, qui renferme au moins un des composés (I), qui sont obtenables par transformation d'au moins un des groupes carboxyle contenus dans les acides gras diméres ou trimères correspondants, en groupes $-CH_2N[CH_2-CH_2-CO_2H]_2$ etlou $-CH_2N[CH_2-CH(CH_3)-CO_2H]_2$, dans lequel on met en oeuvre les composés (I) sous forme d'acide ou de leurs sels de métaux alcalins, de métaux alcalino-terreux, d'ammonium, d'alkylammonium ou d'alcanolammonium, caractérisé en ce que l'on se passe d'une fixation ultérieure par des sels minéraux.

**7.** Agents pour mettre en huile les cuirs, les peaux et les matières similaires, sous la forme d'une solution ou d'une dispersion aqueuse, caractérisés en ce qu'ils renferment des acides aminopropioniques dimères et/ou trimères (I), qui sont obtenables par transformation d'au moins un des groupes carboxyle ($-CO_2H$) contenus dans les acides gras dimères ou trimères correspondants, en groupes $-CH_2N[CH_2-CH_2-CO_2H]_2$ etlou $-CH_2N[CH_2-CH(CH_3)-CO_2H]_2$, en mettant en oeuvre les composés (I) sous forme d'acide ou de leurs sels de métaux alcalins, de métaux alcalino-terreux, d'ammonium, d'alkylammonium ou d'alcanolammonium.